(19) Europäisches Patentamt
European Patent Office
Office européen des brevets

(11) Publication number: **0 102 833**
**B1**

(12) **EUROPEAN PATENT SPECIFICATION**

(45) Date of publication of patent specification: **13.08.86**

(21) Application number: **83305055.2**

(22) Date of filing: **01.09.83**

(51) Int. Cl.⁴: **C 07 C 51/15**, C 07 C 65/05, C 07 C 65/10, C 07 C 65/11, C 07 D 213/803, C 07 D 213/79, C 07 D 215/48

(54) **Carboxylation of metal aryloxides.**

(30) Priority: **02.09.82 US 417452**

(43) Date of publication of application:
**14.03.84 Bulletin 84/11**

(45) Publication of the grant of the patent:
**13.08.86 Bulletin 86/33**

(84) Designated Contracting States:
**AT BE CH DE FR GB IT LI LU NL SE**

(56) References cited:
**EP-A-0 089 564**
**EP-A-0 089 565**
**DE-B-1 493 881**
**DE-C- 536 997**
**US-A-4 171 453**

**CHEMICAL ABSTRACTS, vol. 49, no. 7, April 10, 1955, Columbus, Ohio, USA, O. BAINE et al. "The Kolbe-Schmitt reaction II. The carbonation of phenols"**

(73) Proprietor: **Euroceltique SA**
**122 Boulevard De La Petrusse**
**Luxembourg (LU)**

(72) Inventor: **Ranus, Walter**
**88 Lakeview Terrace**
**Ramsey N.J. 07446 (US)**
Inventor: **Loewenstein, Robert**
**23 Hillcrest Road**
**Belle Mead N.J. 08502 (US)**
Inventor: **Weiberth, Franz**
**91 Spring Street**
**Lodi N.J. 07644 (US)**
Inventor: **Zirlis, Joseph**
**30 Third Avenue**
**Hawthorne N.J. 07506 (US)**
Inventor: **Bugbee, Holbrook**
**3 Fernwood Place**
**Mountain Lakes N.J. 07046 (US)**

(74) Representative: **Lamb, John Baxter et al**
**Marks & Clerk 57/60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention is concerned with improvements in and relating to the carboxylation of metal aryloxides.

Metal aryloxides such as potassium phenoxide are presently carboxylated by loading a ball mill-type reactor with phenol and aqueous potassium hydroxide and evaporating the resulting product to zero moisture under air-tight vacuum. The purpose of the ball mill-type reactor is to produce a fine powder. The reactor is pressurized with carbon dioxide to about 250 lbs (17.6 $Kg/cm^2$) pressure at a temperature range of 180°C to 25°C and maintained at this temperature for 6—12 hours. As indicated, the milling action of the ball mill is necessary for the gas to react with the powder. One of the difficulties of this process is the severe caking which takes place during the reaction.

Also as indicated the reaction requires 6—12 hours and when the reaction is finished the phenol, which is a product of the rearrangement, must be distilled under vacuum. Upon complete distillation the reactor is cooled to permit sufficient water to dissolve the cake-like product, removed from the reactor and transferred to acidification.

In US—A—4 172 453 there is described a two-stage process for the carbonylation of metal aryloxides in which the first stage is carried out at a temperature below 135°C and the second at a temperature above 135°C.

There are described in EP—A—0089564 and EP—A—0089565 processes for the carbonylation of metal aryloxides in a fluidized bed system.

It is a primary object of the present invention to provide for a simpler method of carboxylating metal aryloxides. Although the invention is applicable to a wide range of metal aryloxides, as will be discussed in greater detail below, for the purpose of convenience, the invention will generally be discussed with reference to the carboxylation of potassium phenoxide.

It is another object of the present invention to provide for a method of carboxylating metal aryloxides which avoids the need for a ball mill and which results in the production of a homogeneous product in a dry state.

It is still another object of the present invention to provide for a method of carboxylating metal aryloxides with a minimum production of isomeric and polycarboxylated contamination products.

It is yet another object of the present invention to provide a more rapid method of carboxylating metal aryloxides in high yield.

According to the invention there is provided a one-stage process of carbonylating a metal aryloxide by reacting the metal aryloxide in the fluidized particulate form with carbon dioxide within a predetermined temperature range characterised in that the one-stage carbonylation process is performed entirely under a vacuum pressure between 2.54 and 759 mm of Hg (0.03—1.01 bar) and within a temperature range of from ambient to 288°C.

The method of the present invention is applicable to metal aryloxides in general, and more particularly to the following metal aryloxides:
metal phenoxides of the formula:

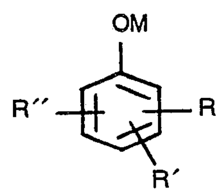

(I)

metal dinuclearphenoxides of the formula:

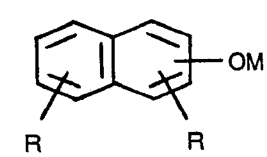

(II)

metal heteroaryloxides of the formula:

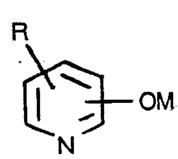

(III)

and metal dinuclearheteroaryloxides of the formula:

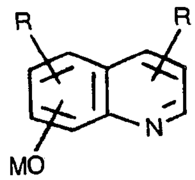

(IV)

wherein

M is an alkali metal such as lithium, sodium, potassium, cesium or rubidium,

R is H, alkyl, halogen, hydroxy or alkoxy;

R' is H, alkyl, halogen, hydroxy or alkoxy; and

R'' is H, alkyl, hydroxy, amino, alkylamino or dialkylamino.

Where the substituent "R" is used more than one time in a formula, then it may be the same or different, e.g. in the metal dinuclearphenoxide one "R" can be hydrogen and the other "R" can be alkyl.

When the term "alkyl" is used, it is generally applicable to all alkyls. However, the lower alkyls containing 1—5 carbon atoms are preferred.

The carboxylation is effected by means of carbon dioxide.

The fluidization of the powdered metal aryloxide can be effected by gaseous means, for example a non-reactive gas or by mechanical means with set or variable speed mixing.

The degree of vacuum can vary widely between 0.1 inch (0.254 cm) mercury to 29.9 inches (75.9 cm) of mercury (0.03—1.01 bar). It is interesting to note that the vacuum is maintained although carbon dioxide is passed into the reaction vessel because the carbon dioxide as it is introduced reacts with the metal aryloxide and is taken out. Non-reactive gas used for fluidization and any non-reacted gas can be recycled.

The carboxylation of the metal aryloxide results in the formation of the salt of the corresponding acid in the form of a dry free flowing powder. The reaction product is free of isomers, free of polycarboxylated material and free of any significant amount of oxidized colour bodies.

The salt can be acidified to form the free acid by conventional methods using as acidification agent a mineral acid or a short chain alkanoic acid.

The salts of the acid can also be esterified with alkyl alcohols of, for example, 1—7 carbon atoms to produce the corresponding esters and their geometric isomers.

The carboxylation of potassium phenoxide and subsequent acidification reaction proceeds as follows:

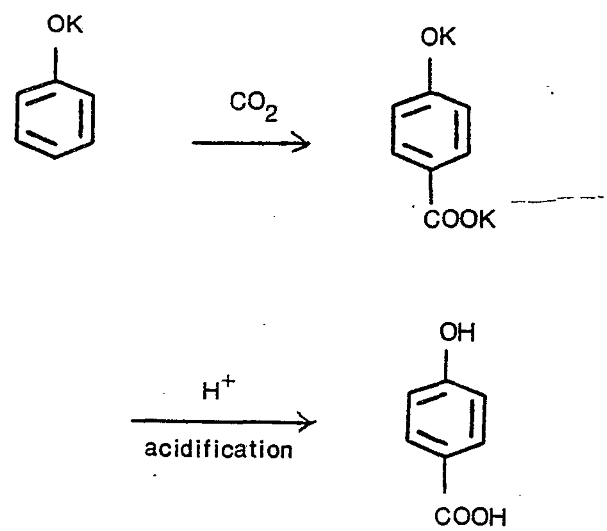


In actual practice, the arylhydroxide is first converted to the metal aryloxide by means of a base, preferably sodium hydroxide or potassium hydroxide. The resulting sodium aryloxide or potassium aryloxide is then carboxylated as indicated above, after which it is acidified to form the desired product.

Thus, for example, the overall process can proceed from the following starting materials to the end products indicated:

| ARYLHYDROXIDE | BASE USED | PRODUCT |
|---|---|---|
| Phenol | NaOH | Salicylic Acid |
| 2,5-dimethyl phenol | NaOH | 3,6-dimethyl-2-hydroxy benzoic acid |
| 3,4-dimethyl phenol | NaOH | 4,5-dimethyl-2-hydroxy benzoic acid |
| 4-chloro phenol | NaOH | 5-chloro-2-hydroxy benzoic acid |
| 3,5-dimethyl phenol | KOH | 2,4-dimethyl-6-hydroxy benzoic acid |
| 2-naphthol | KOH | 2-hydroxy-6-naphtholic acid |
| 6-hydroxyquinoline | KOH | 6-hyroxyquinoline-5-carboxylic acid |
| 2-hydroxycarbazole | KOH | 2-hydroxycarbazole-3-carboxylic acid |

The following examples are given to further illustrate the present invention.

### Example I

Charge a 130 l. horizontal reactor equipped with condenser, receiver, and gas recycle loop with 132 lbs (59.9 Kg) of dry potassium phenoxide. Evacuate the reactor and engage the agitator. Heat the reactor to 340°F (171°C) material temperature. Begin to introduce 22 lbs (9.98 Kg) of carbon dioxide at a rate so as to maintain a constant vacuum. After all the carbon dioxide has been reacted, cool the reactor to 150°F (65.5°C) internal material temperature and discharge the powder.

Wt. of powder collected = 107 lbs. (48.5 Kg)

Wt. of phenol collected = 47 lbs. (21.3 Kg)

TLC analysis reveals 1 detectable spot.

When the product is dissolved in water and is acidified with sufficient concentrated hydrochloric acid to pH of 2, the desired acid is free of isomeric and polycarboxylated material M.P. 213.5—215°C.

### Example II

Charge a 130 l. vertical reactor equipped with condenser, receiver, and gas recycle loop with 132 lbs (59.9 Kg) of dry potassium phenoxide. Evacuate the reactor and introduce a non-reactive gas, preferably $N_2$, through the bottom of the reactor at a rate to fluidize the bed utilizing the gas recycle loop. Heat the reactor to 340°F (171°C) material temperature. Begin introducing 22 lbs (9.98 Kg) of carbon dioxide at a rate so as to maintain constant vacuum. After all the carbon dioxide has been reated, cool reactor to 150°F (65.50°C) material temperature, remove and collect the fluidizing gas and discharge the powder.

Wt. of powder collected = 107 lbs. (48.5 Kg)

Wt. of phenol collected = 47 lbs. (21.3 Kg)

TLC analysis reveals 1 detectable spot.

When the product is acidified as in Example I, then the desired acid (*p*-hydroxy-benzoic acid) is free of isomeric and polycarboxylated material. M.P. 213.5—215°C.

### Example III

Preparation of 2,4-Dimethyl-6-Hydoxy Benzoic Acid

Charge a 130 l. horizontal reactor equipped with condenser, receiver, and gas recycle loop with 72.6 Kg of dry potassium 3,5-dimethyl phenoxide. Evacuate the reactor and engage the agitator. Heat the reactor to 340°F (171°C) material temperature. Begin to introduce 23 lbs (10.43 Kg) of carbon dioxide at a rate so as to maintain constant vacuum. After all the carbon dioxide has been reacted, cool the reactor to 150°F (65.5°C) internal material temperature and discharge the powder. The powder is acidified by conventional methods, the desired acid is free of isomeric and polycarboxylated material. M.P. 166°C.

### Example IV

Preparation of 6-Hydroxyquinoline-5-Carboxylic Acid

Charge a 130 l. horizontal reactor equipped with condenser, receiver, and gas recycle loop with 183 lbs (83 Kg) of dry 6 potassium oxyquinolinate. Evacuate the reactor and engage the agitator. Heat the reactor to 340°F (171°C) material temperature. Begin to introduce 45 lbs (20.41 Kg) of carbon dioxide at a rate so as to maintain constant vacuum. After all the carbon dioxide has been reacted, cool reactor to 150°F (65.5°C) internal material temperature and discharge powder. The powder is acidified by conventional methods, the desired acid is free of isomeric and polycarboxylated material. M.P. 203—204°C.

Example V

Preparation of 2-Hydroxy-6-Naphthoic Acid

Charge:

182 lbs (82.6 Kg) of dry potassium-2-naphthate

23 lbs (10.43 Kg) of carbon dioxide)

Procedure same as Example III. M.P. 245—248°C.

Example VI

Preparation of 2-Hydroxycarbazole-3-Carboxylic Acid

Charge:

222 lbs (100.7 Kg) of potassium-oxycarbazole

44 lbs (19.95 Kg) of carbon dioxide)

Procedure same as Example III. M.P. 273—274°C.

Example VII

(a) Esterification of dipotassium *p*-oxybenzoate yielding methyl p-hydroxybenzoate

Add 78 g of dipotassium-*p*-oxybenzoate to 130 ml. methanol containing 56.0 g of sulphuric acid. Heat under reflux for 18 hours filter and adjust to pH 3.9 with aqua ammonia. Add 100 ml. of $H_2O$, cool, and collect the product. M.P. 125—127°C; Mixed M.P. 125—126°C.

(b) Esterification of dipotassium-*p*-oxybenzoate yielding propyl p-hydroxybenzoate

Add 78 g of dipotassium-*p*-oxybenzoate to 200 ml of propyl alcohol containing 56 g of sulphuric acid. The mixture was heated under reflux for 18 hours, filtered and pH was adjusted to 4 with aqua ammonia. The solution was poured into 100 ml of $H_2O$ and cooled. A solid was collected. M.P. 95—98°C; Mixed M.P. 95—97°C.

Example VIII

Charge a 130 litre horizontal reactor equipped with condenser, receiver and gas recycle loop with 116 lbs (52.6 Kg) of dry sodium phenoxide. Evacuate the reactor and maintain cooling in jacket. Begin to introduce 45 lbs (20.41 Kg) of carbon dioxide at a rate so as to maintain constant vacuum and a temperature of 70°F to 110°F (21—43°C). After all the carbon dioxide has been reacted, discharge the powder. Acidification by conventional methods yields the desired salicylic acid, free of an isomeric and polycarboxylated material. M.P. = 157° to 160°.

Example IX

Preparation of 5-chloro-2-hydroxy benzoic acid

Charge:

150 lbs (68 Kg) of dry sodium 4-chlorophenoxide

45 lbs (20.41 Kg) of carbon dioxide.

Procedure as in Example VIII. M.P. 170°—172°C.

Example X

Preparation of 5-methyl-2-hydroxy benzoic acid

Charge:

130 lbs (59 Kg) of dry sodium 4-methylphenoxide

45 lbs (20.41 Kg) of carbon dioxide.

Procedure as in Example VIII. M.P. 150°—154°C.

**Claims**

1. A one-stage process of carboxylating a metal aryloxide comprising reacting the aryloxide in fluidized particulate form with carbon dioxide within a predetermined temperature range characterised in that the one-stage carbonylation is performed entirely under a vacuum pressure of between 2.54 and 759 mm of mercury (0.03—1.01 bar) and within a temperature range of from ambient to 288°C.

2. A method according to claim 1 wherein the metal is an alkali metal.

3. A method according to claim 1 wherein the metal aryloxide is of the formula:

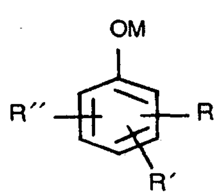

(I)

or

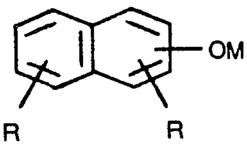

(II)

or

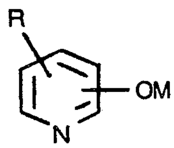

(III)

or

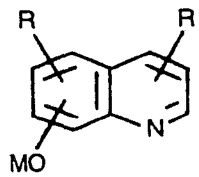

wherein

M is an alkali metal and

R is H, alkyl, halogen, hydroxy or alkoxy;

R′ is H, alkyl, halogen, hydroxy or alkoxy; and

R′′ is H, alkyl, hydroxy, amino, alkyl amino or dialkylamino.

4. A method according to claim 3 wherein the metal aryloxide is an alkali metal phenoxide, alkali metal lower alkyl phenoxide, alkali metal naphthoxide or alkali metal hydroxyquinoline.

5. A method according to claim 4 wherein said aryloxide is potassium phenoxide.

**Patentansprüche**

1. Einstufiges Verfahren zur Carboxylierung von Metallaryloxiden, das die Reaktion des Aryloxides in fluidisierter Partikulatform mit Kohlendioxid innerhalb eines vorgegebenen Temperaturbereiches umfaßt, dadurch gekennzeichnet, daß eine einstufige Carbonylierung vollständig unter Vakuumdruck zwischen 2,54 und 759 mm Quecksilber (0,03—1,01 bar) und innerhalb eines Temperaturbereiches von Raumtemperatur bis 288°C durchgeführt wird.

2. Verfahren nach Anspruch 1, dadurch gekennzeichnet, daß das Metall ein Alkalimetall ist.

3. Verfahren nach Anspruch 1, dadurch gekennzeichnet, daß das Metallaryloxid von der Formel

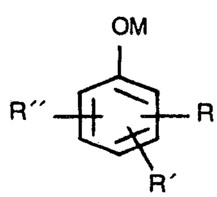

(I)

oder

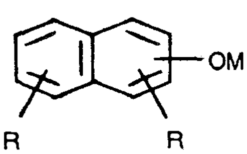

(II)

oder

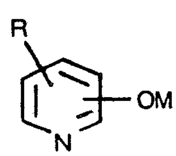

(III)

oder

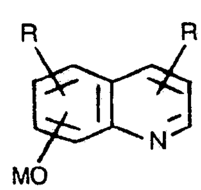

ist, wobei

M ein Alkalimetall ist und

R H, Alkyl, Halogen, Hydroxy oder Alkoxy ist;

R' H, Alkyl, Halogen, Hydroxy oder Alkoxy ist; und

R'' H, Alkyl, Hydroxy, Amino, Alkylamino oder Dialkylamino ist.

4. Verfahren nach Anspruch 3, dadurch gekennzeichnet, daß das Metallaryloxid ein Alkalimetallphenoxid, Alkalimetallphenoxid mit niedriger Alkylgruppe, Alkalimetallnaphthoxid oder Alkalimetallhydroxychinolin ist.

5. Verfahren nach Anspruch 4, dadurch gekennzeichnet, daß Aryloxid Kaliumphenoxid ist.

**Revendications**

1. Un procédé de carboxylation en une étape d'un aryloxyde métallique qui consiste à faire réagir l'aryloxyde sous forme particulaire fluidisée avec du dioxyde de carbone à l'intérieur d'une gamme de température prédéterminé, caractérisé en ce que la carbonylation en une étape est effectuée entièrement sous un vide correspondant à une pression comprise entre 2,54 et 759 mm de mercure (0,03 à 1,01 bar) et dans une gamme de températures allant de l'ambiance à 288°C.

2. Un procédé selon la revendication 1 dans lequel le métal est un métal alcalin.

3. Un procédé selon la revendication 1 dans lequel l'aryloxyde métallique correspond à la formule:

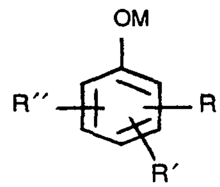

(I)

ou

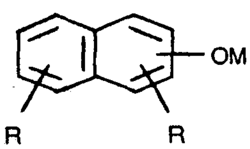

(II)

ou

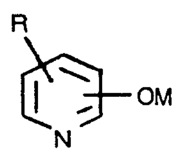

(III)

ou

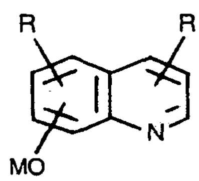

dans laquelle

M est un métal alcalin et

R est H un alcoyle, un halogène, un hydroxyle ou un alcoxyle.

R' est H un alcoyle, un halogène, un hydroxyle ou un alcoxyle et

R'' est H un alcoyle, un hydroxyle, un amino, un alcoyl-amino ou un dialcoylamino.

4. Un procédé selon la revendication 3 dans lequel l'aryloxyde métallique est un phénoxyde de métal alcalin, un (alcoyle inférieur)-phénoxyde de métal alcalin, un naphtoxyde de métal alcalin ou une hydroxyquinoléine de métal alcalin.

5. Un procédé selon la revendication 4 dans lequel ledit aryloxyde est un phénoxyde de potassium.